# EUROPEAN PATENT SPECIFICATION

(11) **EP 3 209 208 B1**
(45) Date of publication and mention of the grant of the patent: **04.12.2019**
(21) Application number: 15787842.2
(22) Date of filing: 21.10.2015
(51) Int. Cl.: A61B 6/00, A61B 6/10

(54) **MOBILE RADIOGRAPHIC IMAGING APPARATUS**
MOBILE RÖNTGENBILDGEBUNGSVORRICHTUNG
APPAREIL D'IMAGERIE RADIOGRAPHIQUE MOBILE

(30) Priority: 22.10.2014 US 201462067083 P
(43) Date of publication of application: 30.08.2017
(73) Proprietor: Carestream Health, Inc., Rochester, NY 14608 (US)
(72) Inventor: FICARRA, Michael, Rochester, NY 14608 (US); OGLE, James, H., Jr., Rochester, NY 14608 (US); WENDLANDT, William, C., Rochester, NY 14608 (US)
(74) Representative: Klang, Alexander H.
(86) International application number: PCT/US2015/056644
(87) International publication number: WO 2016/064993

(56) References cited:
- WO-A1-2013/072810
- DE-A1-102004 042 063
- US-A1- 2007 211 847
- US-B2- 7 597 473

## Description

### FIELD OF THE INVENTION

The invention relates generally to the field of medical imaging, and in particular to a mobile radiographic imaging apparatus. More specifically, the invention relates to a mobile radiography system having a radiation source mounted to an adjustable radiation source support structure.

### BACKGROUND OF THE INVENTION

Mobile carts are employed in medical facilities to move medical equipment between locations. One type of mobile cart may include an x-ray source used to capture digital x-ray images on a digital x-ray detector. Medical x-ray images can be captured using various techniques. For example, techniques such as computed radiography (CR) and digital radiography (DR) can be used to obtain medical images.

Mobile x-ray apparatus are of particular value in various medical environments, such as intensive care units, where timely acquisition of a radiographic image is important. Because portable carts can be wheeled around and brought directly to the patient's bedside, a portable x-ray imaging apparatus allows an attending physician, clinician, or other operator to have recent information on the condition of a patient and helps to reduce the risks entailed in moving patients to stationary equipment in the radiological facility. Such mobile x-ray devices are known from e.g. US-7, 597, 473 B2 or US-2007/0211847 A1, which both show mobile radiography systems with moveable arms and sensors for detecting objects. However, there is a need for improvements in mobile x-ray apparatus design to allow such devices to be more carefully maneuvered or positioned so that unintentional injurious bumping against a patient can easily and automatically be avoided.

The discussion above is merely provided for general background information and is not intended to be used as an aid in determining the scope of the claimed subject matter.

### BRIEF DESCRIPTION OF THE INVENTION

A mobile radiography system includes a moveable arm having an x-ray source attached thereto which can be manually positioned by an operator. A sensor detects a location of a patient or patient's bed relative to the radiography system and generates location information so that a programmable encoder monitors the position and a brake or motor control restricts movement of the arm within a zone proximate the patient. An advantage that may be realized in the practice of some disclosed embodiments of the mobile radiography apparatus is that a margin of safety is provided for the patient and operator during mobile x-ray imaging to prevent impact of the arm of the mobile medical imaging cart with a patient. The system may be applicable to a mobile x-ray system, such as in the cart embodiments disclosed herein or to a stationary system.

In one embodiment, the cart may be transported adjacent to a bed having a patient thereon. The cart, or x-ray source support arm, or both, may have a means for identifying the location of the bed relative to the cart which may be achieved using a mechanical device(s), a sensor(s), or through an operator input to identify the location of the bed. Using the position of the bed relative to the cart, a region designated as a zone may be determined. When the x-ray imaging head is undocked from the cart, the arm may be subsequently prevented from protruding or extending into the zone. This may be achieved using a mechanism(s) which may consist of a brake(s), an encoder(s), a motor controller(s), or similar mechanism(s) to limit travel of the arm, or any components of the arm, from coming into contact with the patient. Such a motion restriction may be applied to any single joint or a multiple set of joints to achieve the desired restriction. The x-ray imaging head portion of the arm may be permitted to move into the zone when the operator is positioning it for a radiographic image acquisition. The restriction may include preventing movement into the zone or it may include reduced speed of movement through the zone.

In another embodiment, a mobile radiography system comprises a moveable arm having an x-ray source attached thereto. The x-ray source is configured to be manually positioned by an operator and includes a sensor for detecting a location of a patient relative to the apparatus and for generating location information corresponding to the location of the patient. The movable arm may be capable of moving laterally toward the patient, however, a mechanism in the system may be activated which is responsive to the location information for controlling movement of the arm with respect to a zone proximate the patient.

In another embodiment, a method of operating a mobile radiography system includes receiving a signal indicating that the mobile radiography system is near a bed or other object. A position of the system is determined at the time of receiving the signal and a logical boundary of a zone is determined, whereby the boundary location is defined as between the system and the bed or other object, and monitored as to whether the system traverses the boundary.

This brief description of the invention is intended only to provide a brief overview of subject matter disclosed herein according to one or more illustrative embodiments, and does not serve as a guide to interpreting the claims or to define or limit the scope of the invention, which is defined only by the appended claims. This brief description is provided to introduce an illustrative selection of concepts in a simplified form that are further described below in the detailed description. This brief description is not intended to identify key features or essential features of the claimed subject matter, nor is it intended to be used as an aid in determining the scope of the claimed subject matter. The claimed subject matter is not limited to implementations that solve any or all disadvantages noted in the background.

For example, the summary descriptions above are not meant to describe individual separate embodiments whose elements are not interchangeable. In fact, many of the elements described as related to a particular embodiment can be used together with, and possibly interchanged with, elements of other described embodiments. Many changes and modifications may be made within the scope of the present invention and the invention includes all such modifications. The drawings below are intended to be drawn neither to any precise scale with respect to relative size, angular relationship, relative position, or timing relationship, nor to any combinational relationship with respect to interchangeability, substitution, or representation of a required implementation.

### BRIEF DESCRIPTION OF THE DRAWINGS

So that the manner in which the features of the invention can be understood, a detailed description of the invention may be had by reference to certain embodiments, some of which are illustrated in the accompanying drawings. It is to be noted, however, that the drawings illustrate only certain embodiments of this invention and are therefore not to be considered limiting of its scope, for the scope of the invention encompasses other equally effective embodiments. The drawings are not necessarily to scale, emphasis generally being placed upon illustrating the features of certain embodiments of the invention. In the drawings, like numerals are used to indicate like parts throughout the various views. Thus, for further understanding of the invention, reference can be made to the following detailed description, read in connection with the drawings in which:
FIG. 1 is a diagram of an exemplary mobile radiography system in a position proximate a bed having a patient thereon with a representation of a zone proximate the patient;
FIG. 2 illustrates an example of a situation where an arm of the mobile radiography system may impact the patient;
FIG. 3 is a schematic diagram of a definition of a zone;
FIG. 4 is a flow chart of an exemplary method of the present invention; and
FIG. 5 illustrates an exemplary cart processing system.

### DETAILED DESCRIPTION OF THE INVENTION

This application claims priority to U.S. Patent Application Serial No. 62/067,083, filed October 22, 2014, in the name of Ficarra et al.

This application is related in certain respects to U.S. Patent No. 8,568,028, issued October 29, 2013, in the name of Wendlandt et al., and entitled MOBILE RADIOGRAPHY UNIT HAVING COLLAPSIBLE SUPPORT COLUMN.

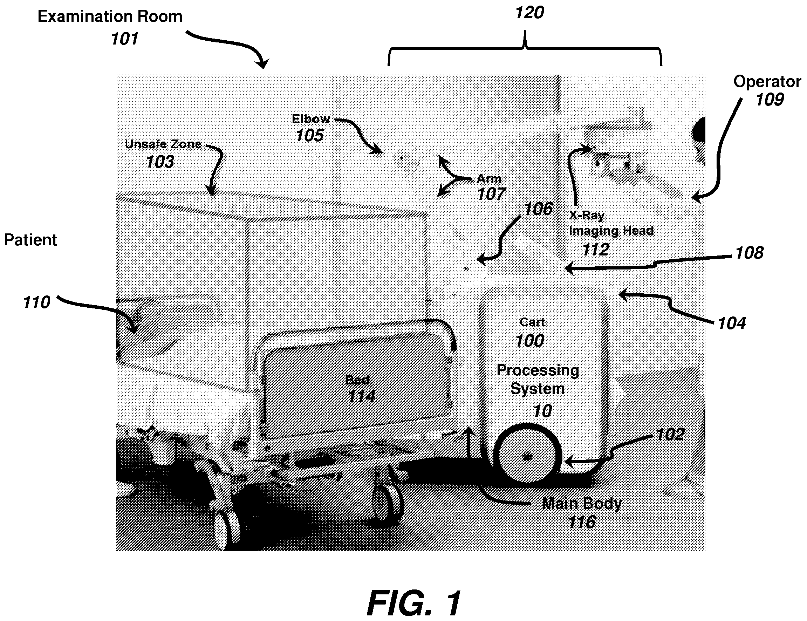

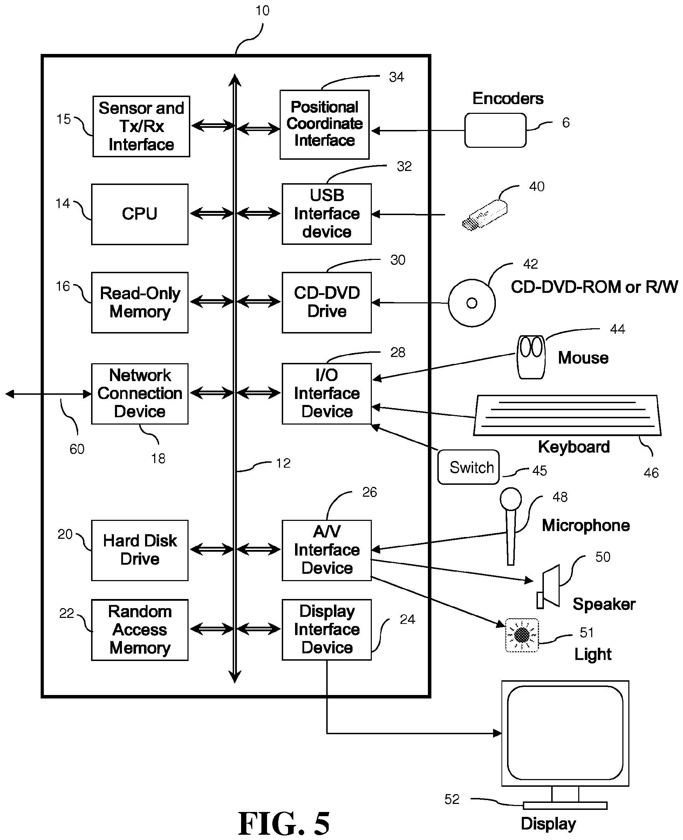

FIG. 1 illustrates an exemplary mobile radiography system 120 that includes a cart 100 having attached thereto, at rotatable joint 106, a segmented movable support arm 107 with rotatable elbow 105 configured to support an x-ray imaging head 112 that may be manually adjusted to any lateral position, height and angle by an operator 109. The cart 100 may be said to have a main body portion 116 with handle 104 and a display device 108, among other components. The mobile radiography system 120 may be used, for example, in a medical facility examination room 101 having a bed 114 supporting a patient 110 thereon. The mobile radiography system 120 is capable of acquiring radiographic (x-ray) images of the patient using the x-ray head 112 attached to the movable arm 107 which itself is attached to the rotatable joint 106 at one end of the support arm 107. A digital radiography detector (not shown) may be positioned under the patient 110, for example, to capture a radiographic image of the patient 110 as the x-ray imaging head 112 is fired. For mobility, the cart 100 has two or more wheels 102 and one or more handles 104 typically provided at about waist level, for use by the operator 109 to guide the mobile radiographic system 120 to its intended location as it is wheeled into position. The mobile radiographic system 120 may include a rechargeable battery pack (not shown) that typically provides source power, which can reduce or eliminate the need for operating the mobile radiographic system 120 near a power outlet. The battery pack may provide for assisted transport by powering a motor for driving the wheels 102, and may also provide power to an on-board cart processing system 10 (FIG. 5) that controls various operations of the mobile radiography system 120, as described herein. Alternatively, the mobile radiographic system 120 may exclude a battery pack and be operable only when connected to an external power source such as an AC power supply or plugged into a power outlet. When used for digital radiography, the cart 100 may include physical storage for holding one or more DR detectors. For example, the cart 100 may include a holder, such as a slot, for holding or storing one or more digital detectors or computed radiography cassettes. The holder may be disposed on the cart 100 and configured to retain at least one digital radiography (DR) detector and/or detectors having different sizes.

Mounted to a frame or housing of the cart 100 is the support arm 107 having an elbow joint 105 or similar structure for allowing three-dimensional movement and positioning of the x-ray imaging head 112 mounted to the support arm 107. In the embodiment shown in FIG. 1, the support arm 107 may include an articulated member that rotates at an elbow 105 to allow movement of the x-ray head 112 over a range of vertical and horizontal positions. In one embodiment, the arm 107 may be rotatably coupled to the cart at one end of the arm 107 using a joint 106 that may allow rotation about two axes, e.g., a horizontal axis and a vertical axis. The joint 106, the arm 107, elbow 105, and x-ray imaging head 112 may be collectively referred to herein as "extensions" to the cart 100. In another exemplary embodiment, the support arm 107 may be constructed using a vertical telescoping section, and a horizontal telescoping section attached to the vertical section for extending the x-ray head outward at a variable distance from the vertical section. Height manipulation for the x-ray imaging head 112 may range from low height for imaging lower extremities to shoulder height and above for imaging the upper body portions of patients in various positions. In exemplary embodiments, the support arm 107 may allow one degree of freedom, two degrees of freedom, or three degrees of freedom for the x-ray head relative to the main body of the cart using the movable support arm 107 such that the x-ray imaging head 112 may be rotated about several axes.

As illustrated in FIG. 1, an exemplary three dimensional zone 103 above the bed 114 and proximate to the patient 110 is illustrated, which zone 103 may represent a spatial region wherein a risk of impact exists as between the patient 110 and parts of the radiographic imaging system 120. The x-ray imaging head 112, the support arm 107, and the elbow 105 of the support arm may present the greatest risk if any of those parts of the radiographic imaging system 120 are maneuvered or extended into the zone 103. Although the zone 103 is depicted in FIG. 1 as a generally rectangular volume above a top surface of the bed 114, the zone 103 may be defined by any shape or region in the examination room 101.

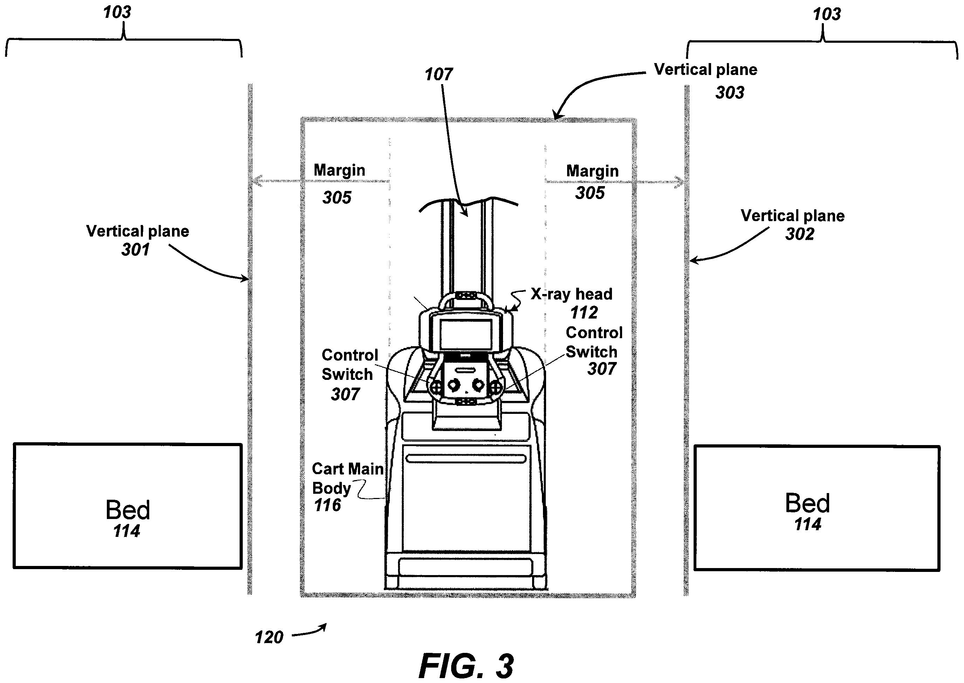

As described herein, the cart 100 may be configured to detect the bed 114 or other object and thereafter programmably react to a spatial region proximate the bed 114 or other object, such as the zone 103 vertically above the bed 114 or other object, and laterally proximate the bed 114 or other object defined by some marginal distance 305 (FIG. 3). As an example, upon detecting an object or a bed 114, the cart processing system 10 may be programmed to define a zone 103 as extending vertically above the detected object, which may encompass space proximate and/or above the detected bed 114 or other object for a predefined distance, such as six feet above a floor, for example.

The exemplary mobile radiographic system 120 may include one or more displays 108, or monitors, located on the main body of the cart 100 and/or on the x-ray imaging head 112. The displays may include manually operable graphical user interface (GUI) controls for operating the mobile radiographic system 120. The mobile radiographic system 120 may further include: a built-in microphone 48 (FIG. 5) for interpreting and initiating responses to a verbal command from the operator 109; a speaker 50 (FIG. 5) for playback of recorded audio messages or for sounding an alarm; a mouse and/or keyboard 44, 46 (FIG. 5) may be provided for operator 109 control over the functions of mobile radiographic system 120. For ease during transport of the cart 100, the support arm 107 and x-ray imaging head 112 may be configured to be foldable against the main body of the cart 100 for additional protection during transport of the cart 100.

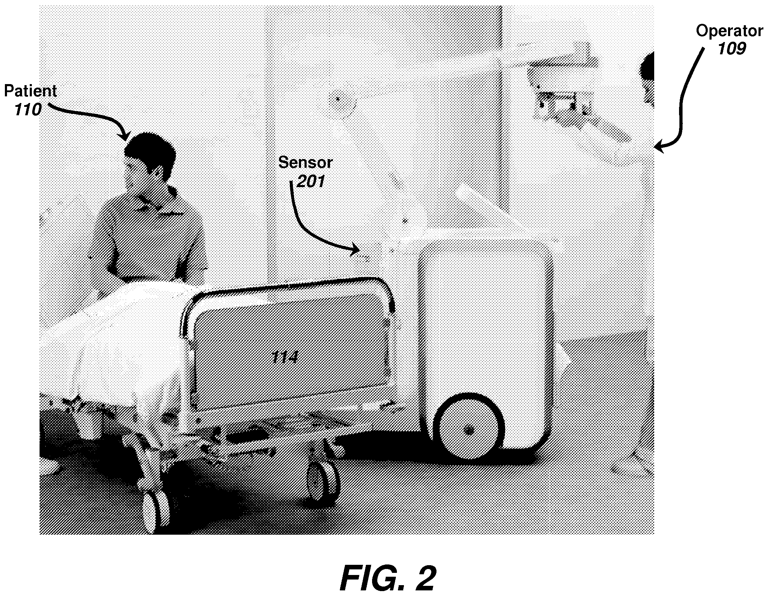

As shown in FIG. 2, the operator 109 may move the support arm 107 during positioning of the x-ray imaging head 112 while the patient 110 is sitting up in the bed 114, whereby either the patient 110, the operator 109, or both may be unaware of the other's movements. As the operator 109 positions the x-ray imaging head 112, the support arm 107 and x-ray imaging head 112 may be extended from the main body of the cart 100. This movement may entail a lateral, e.g., sideways or parallel to the floor, movement of the support arm 107, the x-ray imaging head 112, the elbow 105, or a combination thereof. Such movement may present the risk of an impact between the patient 110 and the support arm 107, x-ray head 112, or the elbow 105.

Still referring to FIG. 2, in one embodiment of the present invention there may be attached to the cart 100 a sensor, or transceiver, 201 that is configured to detect an object, such as a bed 114, proximate to the cart 100. The sensor/transceiver 201 may be a laser based device, an ultrasonic based transceiver device, or electromagnetic based sensor/transceiver, or it may be an infra-red, radio controlled device, an NFC transceiver, or a Bluetooth transceiver, that communicates with another sensor/transceiver mounted on the bed 114. Any such sensor, detector, or transceiver may be configured to be triggered by proximity of the sensor 201 to the bed 114 and to generate a detection signal that is recognized by the cart processing system 10 (FIG. 5), which then may initiate an automatic protection procedure that restricts movement of the cart 100 and its extensions, e.g., the support arm 107, the x-ray imaging head 112, the elbow 105, or a combination thereof, proximate to and/or through the defined zone 103. The detection signal may include location data in its transmission, such as a detected distance between the cart 100 and the bed 114 or other object, so that a zone 103 proximate to a location of the bed 114 or other object relative to the cart 100 may be computed and recognized by the cart's processing system 10. In one embodiment, the cart 100 may make physical contact with the bed 114 or other object. In such an embodiment, the sensor 201 may comprise an accelerometer that detects the physical impact as between the cart 100 and the bed 114 or other object, and transmits the impact detection signal to the cart processing system 10.

At the time when a detection signal is received at the cart processing system 10 from any of the types of sensor 201 described herein, it may be programmed to record data provided thereto by an encoder 6 (FIG. 5) or other components of the mobile radiography system 120. For example, the processing system 10 may access encoder data of an encoder 6 configured to monitor a position of each of two or more wheels 102 to enable the processing system 10 to determine a location of the cart 100 relative to a computed zone 103. The processing system 10 may access encoder data of an encoder 6 configured to monitor an angular position of the joint 106 to enable the processing system to monitor a position of the elbow 105, arms 107, or of the x-ray imaging head 112. The processing system 10 may access encoder data of an encoder 6 at the rotatable elbow 105 configured to monitor an angular gap between the support arms 107 to enable the processing system 10 to monitor with added precision a height position of the of the x-ray imaging head 112. The processing system 10 may be programmed to calculate a position of the cart 100 and its extensions by accessing encoder data at the time of receiving a detection signal, and subsequently determine directional changes in a position of the cart 100 and its extensions relative to zone 103. The processing system 10 may continuously access encoder data corresponding to wheels 102 on opposite sides of the cart 100 to determine a direction and distance that the cart may move along a floor. The processing system 10 may continuously access encoder data corresponding to rotatable joint 106 to determine a rotational angle of the arm 107 relative to the cart 100 and a vertical angle of the lower portion of the arm 107 relative to the cart 100. This data enables computation of a height of the elbow 105, for example. The processing system 10 may continuously access encoder data corresponding to rotatable elbow 105 to determine an angle between the arm 107 upper and lower portions. Taken together with known and electronically stored dimensions of the cart 100, size of the wheels 102, length of the arms 107, and dimensions of the x-ray imaging head, three dimensional spatial positions of the cart 100 and its extensions may be computed by the processing system 10. In one embodiment, the spatial position may be continuously computed by determining a starting position relative to a bed 114 or other object at the time of receiving a detection signal and thereafter continuously monitoring encoder 6 outputs as described herein.

With reference to FIG. 3, there is illustrated an alternative method of operating the radiographic imaging system 120 whereby control switches 307 on the x-ray imaging head 112 (which is shown in a folded position in FIG. 3) or on the main body of the cart 100 may be activated by an operator. For example, if the operator presses the control switch 307 on the right, it may trigger a signal to the processing system 10 that a bed 114 or other object is located to the right of the cart body 116 at a predefined margin or distance 305. Similarly, pressing the control switch 307 on the left may trigger a signal to the processing system 10 that a bed 114 or other object is located to the left of the cart body 116 at a predefined margin or distance 305. Likewise, pressing both control switches 307 may signal the processing system that the bed 114 or other object is in front of the cart body 116. The processing system 10 may also be configured to receive an input from the operator specifying an approximate distance between the cart 100 and the bed 114 or other object. This signal from the operator 109, or a detection signal from sensor 201, may be used by the cart's processing system 10 to define a vertical plane 302 to the right of the cart (which plane extends into and out of the page, as shown) as the spatial border, or boundary, of the zone 103, beyond which movement of the cart or its extensions may be programmably restricted as described herein. The signals from the operator may be used by the cart's processing system 10 to define a vertical plane 301 to the left of the cart (which plane extends into and out of the page, as shown) or a vertical plane 303 in front of the cart, as the spatial borders of a zone 103, beyond which movement of the cart or its extensions may be programmably restricted as described herein. Programming the cart's processing system 10 to recognize various sensor 201 or operator inputs in combination may result in sensing or defining zones 103 proximate to the cart 100 on one or more sides thereof.

Upon receiving a signal from an operator or a detection signal from sensor 201, and in addition to accessing encoder data as described herein, the processing system may be programmed to automatically determine a general or specific location of the bed 114 or other object relative to a location of the cart 100. The processing system 10 may logically define a vertical plane 301-303 between the cart 100 and the bed 114 or other object using internally defined *x-y* coordinates, which vertical plane 301-303 represents a nearest border, or boundary, of the zone 103. The vertical plane 301-303 may itself have preprogrammed dimensions or it may be defined as an infinite plane at a specified distance z from the cart 100. Thus, using an internal *xyz* coordinate system established at a starting time, such as at the time of receiving a detection signal or control signal from the operator, together with the encoder data representing the cart's starting position, the processing system 10 may monitor the spatial position of the cart 100 and its extensions to determine their proximity to the vertical plane 301-303. The processing system 10 may be programmed to prevent the cart 100 and its extensions from moving past the defined vertical plane 301-303 or it may be programmed to restrict movement of the cart 100 or its extensions if the cart or its extensions extend past the vertical plane. The restricted movement may comprise a forced slowing down of movement by the cart 100 or its extensions. In one embodiment, the restricted movement may be effected by electronic motor control signals transmitted to electric motors used to drive the wheels 102, to rotate the joint 106 about either axis, or to rotate the elbow 105. In one embodiment, the restricted movement may be effected by a brake mechanism at the wheels 102, at the joint 106, or at the elbow 105, activated by a control signal from processing system 10 to prevent or restrict movement within zone 103. Other such means may include control of motors, cables, and pulleys used to secure in position the support arm 107 and the x-ray imaging head 112. If movement into the zone is prevented by the processing system 10, the processing system 10 may require the operator 109 to provide particular preselected inputs before the restriction is released, such as by requiring the operator 109 to verify that the patient 110 is in a safe position before proceeding with further positioning of the x-ray imaging head 112.

An encoder 6 (FIG. 5) may be electrically connected to the cart wheels 102, the x-ray imaging head 112, the joint 106, the elbow 105, or a combination thereof, to control movement thereof relative to the zone 103. An encoder 6 may sense a position of a support arm 107 with respect to a rotational position of the arm at the joint 106 that secures the arm 107 to the main body 116 of the cart 100, in addition to an angular position of the arm at the joint 106t. Together with an angle of extension sensed at the elbow joint 105 of the arm, the processing system 10 of the cart 100 may determine where, in three dimensional space, each portion of the support arm 107 is located. Additional position input data may be obtained from an encoder 6 at the x-ray imaging head 112 to define an angle of extension of the x-ray imaging head 112, for example. Additional position input data may be obtained from the wheels 102 of the cart such that the processing system 10 may recognize and record a position of the cart on the examination room floor. Taken together, these positional data may be used by the processing system 10 of the cart 100 to determine a location in space at least of the support arm 107 and the x-ray imaging head 112 in relation to the zone. The positional data may then be used by the processing system 10 to monitor when the cart 100, the support arm 107, and x-ray imaging head 112 are being maneuvered by the operator into, or close to, the zone. Upon detecting such movement, a control sequence may be initiated in the processing system 10 that restricts free movement of the support arm 107 and x-ray imaging head 112 into, proximate to, or through the zone, by transmitting command signals to control motors, cables, brake mechanisms, and/or pulleys embedded therein. Other responses may also be programmed such as activating warning lights or audible warning signals controlled by the cart's processing system 10. Warning light sources and speakers (FIG. 5) may be connected to the processing system and attached to the cart 100, its extensions, or a combination thereof.

The zone 103 may also be logically represented by the processing system 10 as a three dimensional volume of space for controlling movement of the cart 100, the arms 107, and the x-ray imaging head 112 within such a zone 103. A bed 114 detected by a sensor 201 may be a standardized bed so that the processing system may access stored data defining a size and height of the standard bed. Such data may be used by the processing system 10 to further define vertical planes 301-303 at each side of the bed 114 or other object, and their relative location to the cart 100. The zone 103 may be defined to extend a predefined distance above the standard bed, for example. In one embodiment, a standardized bed may include transceivers attached to it at known locations, such as at corners of the bed. The sensor 201 of the mobile radiography system 120 may be able to precisely determine the location of the standard sized bed using a triangulation algorithm with such transceivers.

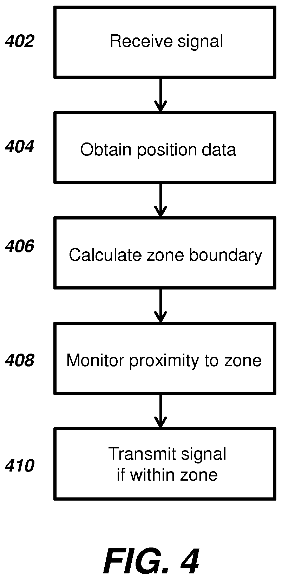

FIG. 4 is a flow chart illustrating a method of operating the mobile radiography system 120 as described herein. At step 402 a signal is received at the processing system 10 of the mobile radiography system 120, which signal may include a detection signal from the sensor 201 or it may include a control switch signal activated by an operator of the mobile radiography system 120. In either case, the signal indicates to the processing system 10 a location of a bed or other object relative to the mobile radiography system 120. At step 404, the processing system may access encoder data defining a position of the wheels 102, the joint 106, the elbow 105, or a combination thereof, in order to determine a position and a location of the cart 100 and its extensions. At step 406, the processing system 10 then computes a location of a zone bounded by at least one vertical plane logically defined by the processing system to be located between the mobile radiography system 120 and the bed 114 or other object. At step 408, the processing system may continuously access encoder data to monitor a current position of the wheels 102, the joint 106, the elbow 105, or a combination thereof, in order to determine if the logically defined vertical plane 301-303 is traversed by any portion of the mobile radiography system 120 including the cart 100 and its extensions. At step 410, if the processing system 10 determines that the boundary of the zone is traversed, it may transmit a signal to mechanically restrict or prevent movement of any part of the mobile radiography system 120 within the zone, or to activate an audio or visual notification, or both.

FIG. 5 illustrates an example mobile radiography processing system 10 useful for practicing embodiments of the present invention. The processing system includes a central processing unit (CPU) 14 that exchanges data electronically with various components, as described herein, over a communication channel 12. The communication channel 12 may include a bus or other wired connection between components, including the CPU 14, and it may, in some instances, include a wireless connection between certain components, as desired. In one embodiment, the mobile radiography system 120 may include input devices such as a keyboard 46, mouse 44, and control switch 307 input 45 electronically connected to the CPU 14 via the processing system's I/O interface device 28. While the keyboard 46 and mouse 44 are illustrated as separate components in FIG. 5, they may be embodied in a GUI presented on a display 52, which display 52 may include a touch sensitive display 52 communicating with CPU 14 over the channel 12 via a display interface 24.

Information from the processing system 10 may be presented on a display 52 mounted on the cart 100 or on the x-ray imaging head 112, or both. Internally, the processing system 10 contains processing system-accessible memory, such as electronic read-only memory 16, random access memory 22, and a hard disk drive 20, which stores programs for performing the functions of the mobile radiography system described herein. Processing system 10 accessible memory may include any processing system-accessible data storage device, whether volatile or nonvolatile, electronic, magnetic, optical, or otherwise, including but not limited to, floppy disks, hard disks, Compact Discs, DVDs, flash memories, such as USB compliant thumb drives, for example.

In addition to fixed media such as a hard disk drive 20, the processing system 10 may also contain processing system-accessible memory drives for reading and writing data from removable processing system-accessible memories. A compact disc/DVD drive 30 may be provided to receive and store programs in the processing system 10 recorded on compatible optical disc media 42, or a USB interface 32 may be provided to receive and store programs in the processing system 10 recorded on USB compatible thumb drive 40. The CD/DVD and USB interface devices may be communicatively connected to the processing system 10 to transfer digital data objects from a device 42, 40 to the processing system's hard disk drive 20 and vice-versa. The CPU 14 may execute software programs stored on, for example, hard disk drive 20 using, as necessary, RAM 22, for example. Audio data may be input, or recorded, in processing system 10 through a microphone 48 communicatively connected to an audio/visual interface device 26. Audio playback such as recorded audio notifications described herein can be played back under program control and heard via a speaker 50 also communicatively connected to an audio/visual interface device 26.

The processing system 10 may activate a light 51 under program control to notify an operator of the mobile radiography system 120. The processing system 10 can be communicatively connected to an external network 60 via a network connection device 18, thus allowing the processing system 10 to access digital data, programs, and digital object from other processing systems, devices, or data-storage systems communicatively connected to the network. Software for programmably operating the mobile radiography system 120 as described herein may be loaded into processing system 10, e.g., on the hard disk drive 20, using CD/DVD media 42, thumb drive media 40, or from remote data storage devices, such as a networked hard drive accessible via the network 60.

The sensor 201 of the mobile radiography system 120 may communicate detection signals to the processing system 10 via transceiver interface 15. Transceiver interface 15 may be used by the processing system 10 to wirelessly communicate with other transceivers situated in an examination room 101 as described herein, for example to communicate position and location information corresponding to the bed 114 or other objects in the examination room 101. The processing system 10 may include Bluetooth compliant firmware, for example, for communicating with a Bluetooth transceiver mounted on the bed 114 via the transceiver interface 15. Encoders 6 located at the wheels 102, joint 106, elbow 105, as described herein, may communicate with processing system 10 via the positional coordinate interface 34 using a wired or wireless connection.

### ALTERNATIVE EMBODIMENTS

In one alternative embodiment, motor control of the wheels 102 of the cart 100 may be employed to restrict movement of the cart toward the zone, in addition to the features described herein with respect to restricting movement of the support arm into the zone. For example, the wheels 102 of the cart 100 may be automatically locked under control of the processing system 10 to prevent movement of the cart 100 along an examination room floor.

In another alternative embodiment, the cart 100 may be programmed into a default inoperative state until the operator 109 activates one of the control switches 307 (FIG. 3) to begin an image acquisition. Similarly, if the elbow 105, or joint 106, encoder senses that an operator 109 is moving the support arm 107, and a control switch 307 has not been pressed, or a sensor 201 has not yet detected, or the system 120 has not yet established, the location of a zone 103, then an automatic shutdown or movement restriction may be triggered. After a location of the zone 103 is determined, either by operator 109 input or by automatic sensor 201 input, then the restricted movement of the support arm 107 may be released. This type of automatic controlled shutdown may be used as a signal to the operator 109 that a sensor 201 is blocked from sensing the presence of a bed, for example, or that the sensor, or another device, is otherwise inoperative. Any such default restriction, or deactivation mechanism, described herein may be nullified by providing an override control that may be activated by the operator.

As will be appreciated by one skilled in the art, aspects of the present invention may be embodied as a system, method, or computer program product. Accordingly, aspects of the present invention may take the form of an entirely hardware embodiment, an entirely software embodiment (including firmware, resident software, micro-code, etc.), or an embodiment combining software and hardware aspects that may all generally be referred to herein as a "system." Furthermore, aspects of the present invention may take the form of a computer program product embodied in one or more computer readable media having computer readable program code embodied thereon. Program code and/or executable instructions embodied on a computer readable medium may be transmitted using any appropriate medium, including but not limited to wireless, wireline, optical fiber cable, RF, etc., or any suitable combination of the foregoing.

Computer program code for carrying out operations for aspects of the present invention may be written in any combination of one or more programming languages, including an object oriented programming language and conventional procedural programming languages. These computer program instructions may also be stored in a computer readable medium that can direct a computer, other programmable data processing system, or other devices to function in a particular manner, such that the instructions stored in the computer readable medium produce an article of manufacture including instructions which implement the function/act specified in the flowchart and/or block diagram block or blocks. The computer program instructions may also be loaded onto a computer, other programmable data processing system, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A mobile radiography system comprising:
a wheeled cart (100),
a moveable arm (107) attached to the wheeled cart and having an x-ray source (112) attached thereto, the x-ray source configured to be positioned by an operator; and
a sensor (201) for detecting a location of a bed (114) or other object relative to the apparatus and for generating location information corresponding to the location of the bed or other object,
wherein the system includes a mechanism responsive to the location information for controlling movement of the arm (107) or the cart (100) with respect to a zone (103) proximate the bed (114) or other object, the mechanism comprising a braking mechanism for preventing the movement of the arm (107) and the cart (100) into the zone (103).

2. The system of claim 1, wherein the mechanism is configured to prevent any portion of the arm (107) and the x-ray source (112) from moving into the zone (103).

3. The system of any of claim 1, wherein the mechanism is configured to forcibly slow movement of the arm (107) if a portion of the arm (107) is moved into the zone (103).

4. The system of claim 1, wherein the system includes at least one of a light source (51) and a sound source (50) that is configured to be activated by the system when the sensor (201) detects movement of the arm (107) or other portion of the system into the zone (103).

5. The system of claim 4, wherein the activated sound source (50) is configured to play a prerecorded message.

6. The system of claim 1, wherein the sensor (201) is selected from the group consisting of a mechanical based sensor, an ultrasonic based sensor, a laser based sensor, an optical sensor, an NFC transceiver, a Bluetooth transceiver, and an electromagnetic wave based sensor.

7. The system of claim 1, wherein the zone (103) comprises a three dimensional space above the bed (114) or other object.

8. The system of claim 1, wherein the zone (103) is a logically defined space on one side of a vertical plane (301, 302), and wherein the mobile radiography system is located on a side of the vertical plane opposite the (103).

9. The system of claim 8, further comprising an input mechanism wherein an operator can select a side of the mobile system to define a position of the vertical plane (301, 302).

10. The system of claim 1, further comprising a communication transceiver positioned proximate the bed (114) or other object, wherein communication between the transceiver and the sensor (201) defines the zone (103).

11. The system of claim 1, further comprising a predefined three dimensional map of an examination room (101) stored in an electronic memory of the system.

12. A method of operating a mobile radiography system comprising a wheeled cart with a movable arm attached, the method comprising:
receiving (402) a signal indicating that the mobile radiography system is near a bed or other object;
determining (404) a position of the system at the time of receiving the signal;
determining (406) a logical boundary of a zone, the boundary between the system and the bed or other object;
determining (408) if movement of the system traverses the boundary; and
activating a brake mechanism to prevent the movement of the system that traverses the boundary.

13. The method of claim 12, further comprising activating a notification system in response to determining that the movement of the system traversed the boundary.

## Patentansprüche

1. Mobiles Radiographiesystem, das Folgendes aufweist:
einen fahrbaren Wagen (100);
einen beweglichen Arm (107), der an dem fahrbaren Wagen befestigt ist und eine daran befestigte Röntgenquelle (112) aufweist, wobei die Röntgenquelle konfiguriert ist, um von einem Bediener positioniert zu werden; und
einen Sensor (201) zum Detektieren einer Position eines Bettes (114) oder eines anderen Objekts in Bezug auf die Vorrichtung und zum Erzeugen von Standortinformationen, die der Position des Bettes oder eines anderen Objekts entsprechen,
wobei das System einen Mechanismus aufweist, der auf die Standortinformationen anspricht, um die Bewegung des Arms (107) oder des Wagens (100) in Bezug auf eine Zone (103) in der Nähe des Bettes (114) oder des anderen Objekts zu steuern, wobei der Mechanismus einen Bremsmechanismus aufweist, um die Bewegung des Arms (107) und des Wagens (100) in die Zone (103) zu verhindern.

2. System nach Anspruch 1, wobei der Mechanismus konfiguriert ist, um zu verhindern, dass sich ein Teil des Arms (107) und der Röntgenquelle (112) in die Zone (103) bewegt.

3. System nach Anspruch 1, wobei der Mechanismus konfiguriert ist, um die Bewegung des Arms (107) zwangsweise zu verlangsamen, wenn ein Abschnitt des Arms (107) in die Zone (103) bewegt wird.

4. System nach Anspruch 1, wobei das System wenigstens eine Lichtquelle (51) und/oder eine Schallquelle (50) aufweist, die konfiguriert ist, um vom System aktiviert zu werden, wenn der Sensor (201) eine Bewegung des Arms (107) oder eines anderen Teils des Systems in die Zone (103) detektiert.

5. System nach Anspruch 4, wobei die aktivierte Schallquelle (50) konfiguriert ist, um eine vorab aufgezeichnete Nachricht abzuspielen.

6. System nach Anspruch 1, wobei der Sensor (201) ausgewählt ist aus der Gruppe bestehend aus einem mechanisch basierten Sensor, einem ultraschallbasierten Sensor, einem laserbasierten Sensor, einem optischen Sensor, einem NFC-Sender-Empfänger, einem Bluetooth-Sender-Empfänger und einem elektromagnetischen wellenbasierten Sensor.

7. System nach Anspruch 1, wobei die Zone (103) einen dreidimensionalen Raum über dem Bett (114) oder dem anderen Objekt aufweist.

8. System nach Anspruch 1, wobei die Zone (103) ein logisch definierter Raum auf einer Seite einer vertikalen Ebene (301, 302) ist, und wobei das mobile Radiographiesystem auf einer Seite der vertikalen Ebene gegenüber der Zone (103) angeordnet ist.

9. System nach Anspruch 8, das ferner einen Eingabemechanismus aufweist, wobei ein Bediener eine Seite des mobilen Systems auswählen kann, um eine Position der vertikalen Ebene (301, 302) zu definieren.

10. System nach Anspruch 1, das ferner einen Kommunikations-Sende-Empfänger aufweist, der in der Nähe des Bettes (114) oder des anderen Objekts angeordnet ist, wobei die Kommunikation zwischen dem Sende-Empfänger und dem Sensor (201) die Zone (103) definiert.

11. System nach Anspruch 1, das ferner eine vordefinierte dreidimensionale Karte eines Untersuchungsraums (101) aufweist, die in einem elektronischen Speicher des Systems gespeichert ist.

12. Verfahren zum Betreiben eines mobilen Röntgensystems, das einen fahrbaren Wagen mit einem daran befestigtem beweglichen Arm aufweist, wobei das Verfahren Folgendes aufweist:
Empfangen (402) eines Signals, das anzeigt, dass sich das mobile Röntgensystem in der Nähe eines Bettes oder eines anderen Objekts befindet;
Bestimmen (404) einer Position des Systems zum Zeitpunkt des Empfangs des Signals;
Bestimmen (406) einer logischen Grenze einer Zone, der Grenze zwischen dem System und dem Bett oder dem anderen Objekt;
Bestimmen (408), ob die Bewegung des Systems die Grenze überschreitet; und
Aktivieren eines Bremsmechanismus, um die Bewegung des Systems zu verhindern, das die Grenze überschreitet.

13. Verfahren nach Anspruch 12, das ferner das Aktivieren eines Benachrichtigungssystems ansprechend auf das Bestimmen, dass die Bewegung des Systems die Grenze überschritten hat, aufweist.

## Revendications

1. Système de radiographie mobile, comprenant :
un chariot sur roues (100),
un bras mobile (107) fixé au chariot sur roues et ayant une source de rayons X (112) fixée audit bras, la source de rayons X étant configurée pour être positionnée par un opérateur ; et
un capteur (201) pour détecter un emplacement d'un lit (114) ou d'un autre objet par rapport à l'appareil et pour générer des informations de localisation correspondant à l'emplacement du lit ou dudit autre objet,
dans lequel le système comporte un mécanisme sensible aux informations de localisation pour commander le mouvement du bras (107) ou du chariot (100) par rapport à une zone (103) proche du lit (114) ou dudit autre objet, le mécanisme comprenant un mécanisme de freinage pour éviter le mouvement du bras (107) et du chariot (100) dans la zone (103).

2. Système selon la revendication 1, dans lequel le mécanisme est configuré pour empêcher toute partie du bras (107) et de la source de rayons X (112) de bouger dans la zone (103).

3. Système selon la revendication 1, dans lequel le mécanisme est configuré pour ralentir de force le mouvement du bras (107) si une partie du bras (107) est déplacée dans la zone (103) .

4. Système selon la revendication 1, dans lequel le mécanisme comporte au moins une source parmi une source lumineuse (51) et une source sonore (50) qui est configurée pour être activée par le système quand le capteur (201) détecte le mouvement du bras (107) ou d'une autre partie du système dans la zone (103).

5. Système selon la revendication 4, dans lequel la source sonore activée (50) est configurée pour lire un message préenregistré.

6. Système selon la revendication 1, dans lequel le capteur (201) est sélectionné dans le groupe constitué d'un capteur à base mécanique, d'un capteur à ultrasons, d'un capteur à base de laser, d'un capteur optique, d'un émetteur-récepteur NFC, d'un émetteur-récepteur Bluetooth, et d'un capteur à base d'ondes électromagnétiques.

7. Système selon la revendication 1, dans lequel la zone (103) comprend un espace tridimensionnel au-dessus du lit (114) ou dudit autre objet.

8. Système selon la revendication 1, dans lequel la zone (103) est un espace défini logiquement sur un côté d'un plan vertical (301, 302), et dans lequel le système de radiographie mobile est situé sur un côté du plan vertical opposé à la zone (103) .

9. Système selon la revendication 8, comprenant en outre un mécanisme d'entrée dans lequel un opérateur peut sélectionner un côté du système mobile pour définir une position du plan vertical (301, 302).

10. Système selon la revendication 1, comprenant en outre un émetteur-récepteur de communication placé à proximité du lit (114) ou dudit autre objet, dans lequel une communication entre l'émetteur-récepteur et le capteur (201) définit la zone (103).

11. Système selon la revendication 1, comprenant en outre une carte tridimensionnelle prédéfinie d'une salle d'examen (101) stockée dans une mémoire électronique du système.

12. Procédé de fonctionnement d'un système de radiographie mobile comprenant un chariot sur roues avec un bras mobile fixé au chariot, le procédé comprenant les étapes suivantes :
la réception (402) d'un signal indiquant que le système de radiographie mobile est à proximité d'un lit ou d'un autre objet ;
la détermination (404) d'une position du système au moment de la réception du signal ;
la détermination (406) d'une frontière logique d'une zone, la frontière entre le système et le lit ou ledit autre objet ;
la détermination (408) de si un mouvement du système traverse la frontière ; et
l'activation d'un mécanisme de freinage pour éviter le mouvement du système qui traverse la frontière.

13. Procédé selon la revendication 12, comprenant en outre l'activation d'un système de notification en réponse à la détermination que le mouvement du système a traversé la frontière.
